# EUROPEAN PATENT SPECIFICATION

(11) **EP 4 099 241 B1**
(45) Date of publication and mention of the grant of the patent: **01.04.2026**
(21) Application number: 21767302.9
(22) Date of filing: 04.03.2021
(51) Int. Cl.: G06Q 10/0639, G06Q 10/08, G06K 19/06

(54) **MANUFACTURING METHOD FOR SENSOR TRANSMITTER FOR CONTINUOUS GLUCOSE MONITORING**
HERSTELLUNGSVERFAHREN FÜR EINEN SENSORSENDER ZUR KONTINUIERLICHEN GLUCOSEÜBERWACHUNG
PROCÉDÉ DE FABRICATION D'UN ÉMETTEUR DE CAPTEUR POUR LA SURVEILLANCE CONTINUE DU GLUCOSE

(30) Priority: 11.03.2020 KR 20200030306
(43) Date of publication of application: 07.12.2022
(73) Proprietor: i-Sens, Inc., Seoul 06646 (KR)
(72) Inventor: LEE, Jin Won, Seoul 06646 (KR)
(74) Representative: BCKIP Part mbB
(86) International application number: PCT/KR2021/002680
(87) International publication number: WO 2021/182797

(56) References cited:
- WO-A2-2017/040700
- KR-A- 20150 130 132
- KR-A- 20180 102 714
- KR-A- 20180 132 555
- KR-A- 20190 098 444
- US-A1- 2010 230 285
- US-A1- 2019 117 133

## Description

### TECHNICAL FIELD

The present disclosure relates to a method of manufacturing a sensor transmitter for continuous blood glucose measurement, and more particularly, relates to a method of manufacturing a sensor transmitter for continuous blood glucose measurement, the method in which, by generating quality management information mapping production information of a sensor and production information of a transmitter used in the sensor transmitter to each other during an assembly process of the sensor transmitter and storing and managing the generated the quality management information in a server, a distribution path or a sales route of the sensor transmitter can be monitored, quality control of the sensor transmitter can be managed, and defective sensor transmitter can be easily tracked using the quality management information.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes need to constantly measure blood glucose for management, so the demand for devices related to blood glucose measurement is steadily increasing. It has been confirmed through various studies that, when diabetic patients strictly control the management of blood glucose, the incidence of complications of diabetes is significantly reduced. Accordingly, it is very important for diabetic patients to measure blood glucose regularly for blood glucose management.

In general, a blood-collecting type blood glucose meter (finger prick type method) is mainly used for blood glucose control in diabetic patients. This blood-collecting type blood glucose meter helps diabetic patients to manage their blood glucose, but because only the result at the time of measurement is displayed, there is a problem that it is difficult of precisely monitoring the blood glucose level that changes frequently. In addition, since the blood-collecting type blood glucose meter needs to collect blood every time to measure blood glucose frequently during the day, there is a problem in that the burden of blood collection is huge for diabetic patients.

Diabetics patients generally experience hyperglycemia and hypoglycemia, and an emergency may occur in the hypoglycemic conditions. Hypoglycemia occurs when sugar content is not kept for a long time, and the patients may become unconscious or die in a worst case. Accordingly, rapid discovery of the hypoglycemic condition is critically important for diabetics. The blood-collecting type blood glucose meter intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

The continuous glucose monitoring system includes a sensor transmitter configured to be attachable to a body part of a user and generate blood glucose biometric information by extracting body fluid, a communication terminal configured to calculate a blood glucose level from the received blood glucose biometric information and output it, and so on. The sensor transmitter has a sensor for continuous blood glucose measurement, a part of the senor inserted into the body, and the sensor extracts the body fluid of the user for a certain period, for example, fifteen (15) days, in a status that the sensor is inserted to the human body. The sensor transmitter periodically generates blood glucose information from the extracted body fluid, and a blood glucose management application is installed to the communication terminal to periodically receive the blood glucose biometric information from the sensor transmitter, calibrate the received the blood glucose biometric information and output it to the user.

Here, the sensor transmitter is an assembly of a sensor and a transmitter for continuous blood glucose measurement, and is manufactured by assembling the sensor and transmitter during the manufacturing process.

Even if the sensor for continuous blood glucose measurement is produced in the same factory, initial calibration information such as sensor sensitivity, offset and so on is different depending on the production process or the manufacturing process of the sensor, and therefore when the user attaches the sensor transmitter to the body and then measures blood glucose biometric information, the sensor must be calibrated first using the initial calibration information.

In general, after activating the blood glucose management application, the user directly inputs the initial calibration information printed on the packaging of the sensor transmitter into the communication terminal to perform the initial calibration of the sensor, and there is a problem in that it is inconvenient for the elderly or users unfamiliar with the blood glucose management application to input the initial calibration information, and accurate blood glucose values may not be acquired due to the input of incorrect initial calibration information or the mistake made during the input process.

On the other hand, the sensor transmitter is manufactured by assembling the sensor and the transmitter, and if defect of some of sensors or transmitters occurs during the manufacturing process, whether there are sensor transmitters having those sensors or transmitters using the same components needs to be checked and defective sensor transmitters should be recalled or users should be notified to discontinue the use of the defective sensor transmitters US 2019/117133 A1 describes a prior art method of manufacturing a sensor including a step of providing an analyte sensor having an elongated body, a first electrode, a second electrode coaxially located within the first electrode, and at least two electrical contacts longitudinally aligned and spaced along a longitudinal axis of the sensor. US 2010/230285 A1 describes prior art glucose monitoring devices which do not require user calibration after in vivo positioning of the devices in the user.

### DETAILED DESCRIPTION OF DISCLOSURE

### Technical Problem

To solve the problem of the conventional art, the purpose of the present disclosure may be for providing a method of manufacturing a sensor transmitter for continuous blood glucose measurement, the method which can generate quality management information mapping production information of a sensor and production information of a transmitter used in the sensor transmitter to each other during an assembly process of the sensor transmitter and use the quality management information.

Another purpose of the present disclosure is for providing a method of manufacturing a sensor transmitter for continuous blood glucose measurement, the method which generates a quality management code configured to be readable of quality management information consisting of product information about a sensor of a sensor transmitter and production information about a transmitter, and attaches a label having the quality management code to an applicator of the sensor transmitter or to an inner packaging or an outer packaging in order to easily input calibration information of the sensor through the quality management code to a communication terminal.

### Solution to Problem

To accomplish the purpose of the present disclosure, a method of manufacturing a sensor transmitter for continuous blood glucose is defined by claim 1. Preferred embodiments are defined in the dependent claims 2-8.

Preferably, the method of manufacturing the sensor transmitter according to an embodiment of the present disclosure further comprise: generating a quality management code used to read the quality management information, and generating a label including the generated quality management code.

Here, the quality management code is used to be capable of reading the production information of the transmitter only among the quality management information, or is used to be capable of reading both the production information of the transmitter and the production information of the sensor.

Here, the label is attached to at least one of an applicator to which the sensor transmitter is mounted, an inner packaging of the applicator, and an outer packaging of the applicator.

Preferably, the method of manufacturing the sensor transmitter further comprises storing the quality management information to memory of the transmitter, wherein the production information of the transmitter among the quality management information is stored to a first area of the memory, and the production information of the sensor among the quality management information is stored to a second area of the memory.

Here, the production information of the sensor is acquired by reading a sensor identifier formed on the sensor.

In an embodiment of the present disclosure, the production information of the transmitter is acquired by reading a transmitter identifier formed on an outer housing of the transmitter.

In another embodiment of the present disclosure, the production information of the transmitter is stored in the memory of the transmitter, and the production information of the transmitter is acquired from the memory.

### Advantageous Effects of Invention

A method of manufacturing a sensor transmitter according to the present disclosure has the following effects.

A method of manufacturing a sensor transmitter for continuous blood glucose measurement according to the present disclosure, by generating quality management information mapping production information of a sensor and production information of a transmitter used in the sensor transmitter to each other during an assembly process of the sensor transmitter and storing and managing the generated the quality management information in a server, can monitor a distribution path or a sales route of the sensor transmitter, manage quality control of the sensor transmitter, and easily track defective sensor transmitter using the quality management information.

Additionally, a method of manufacturing a sensor transmitter for continuous blood glucose measurement according to the present disclosure generates a quality management code configured to be readable of quality management information consisting of product information about a sensor of a sensor transmitter and production information about a transmitter, and attaches a label having the quality management code to an applicator of the sensor transmitter or to an inner packaging or an outer packaging so that the user can easily input various sensor information and transmitter information through the quality management code to a blood glucose management application of a communication terminal.

### BRIEF DESCRIPTION OF THE DRAWINGS

FIG. 1 is a schematic diagram for illustrating a continuous blood glucose measurement system according to an embodiment of the present disclosure.
FIGS. 2 and 3 are diagrams for explaining a process of attaching a sensor transmitter to body using an applicator.
FIG. 4 is a diagram for explaining a process of manufacturing a sensor transmitter according to an embodiment of the present disclosure.
FIG. 5 shows an example of a sensor and a transmitter contained in the tray (230).
FIG. 6 is a diagram for explaining a message transmitted and received between a manufacturing terminal, a management server and a communication terminal according to an embodiment of the present disclosure.
FIG. 7 shows an example of quality management information stored in a management server according to an embodiment of the present disclosure.
FIG. 8 is a functional block diagram for illustrating an example of a manufacturing terminal according to the present disclosure.
FIG. 9 is a flowchart for explaining a method of manufacturing a sensor transmitter according to an embodiment of the present disclosure.

### DESCRIPTION OF EMBODIMENTS OF DISCLOSURE

The technical terms used in the present disclosure are only for the purpose of describing exemplary embodiments, and they are not intended to limit the present which is defined by the appended claims. Also, unless otherwise defined, all technical terms used herein should be construed as having the same meaning as commonly understood by those skilled in the art, and should not be interpreted as being excessively inclusive or excessively restrictive.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

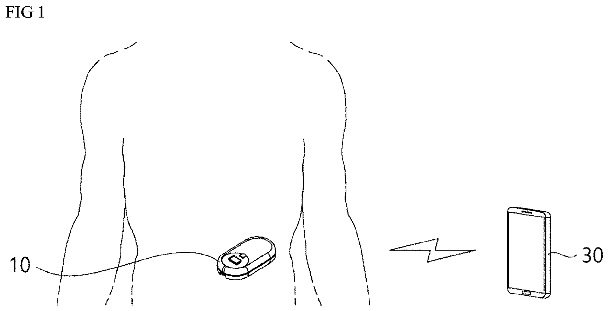

FIG. 1 is a schematic diagram for illustrating a continuous blood glucose measurement system according to an embodiment of the present disclosure.

Referring to FIG. 1, a continuous blood glucose measurement system (1) according to an embodiment of the present disclosure comprises a sensor transmitter (10) and a communication terminal (30).

The sensor transmitter (10) is attachable to human body and, when the sensor transmitter (10) is attached to the human body, an end portion of a sensor of the sensor transmitter (10) is inserted into skin to periodically extract body fluid of the human body and measure blood glucose. Here, the sensor transmitter includes a sensor configured to inserted into the skin to extract a body fluid, and a transmitter configured to measure blood glucose biometric information from the body fluid and transmit the blood glucose biometric information to the communication terminal (30). In the factory, the sensor and the transmitter are assembled together and manufactured as a sensor transmitter, and the sensor transmitter is mounted to an applicator (not shown) configured to attach the sensor transmitter to the body.

After the user takes out the applicator from a packaging and places the applicator on the body part to which the sensor transmitter is to be attached, the sensor transmitter is placed on the skin using the applicator.

After the placement of the sensor transmitter (10) on the skin is completed, the sensor transmitter (10) and the communication terminal communicate with each other to transmit and receive blood glucose biometric information measured by the sensor transmitter (10).

In order to communicationally connect the sensor transmitter (10) and the communication terminal (30) to each other, after activating a blood glucose management application installed in the communication terminal (30), the user acquires connection information by reading a quality management code of a label attached to the applicator or the packaging and automatically connects the communication between the sensor transmitter (10) and the communication terminal (30) based on the acquired connection information. Here, a serial number of the sensor transmitter, a PIN code, etc. may be used as the connection information.

The communication terminal (30) periodically receives blood glucose biometric information from the sensor transmitter (10) after the communication with the sensor transmitter (10) is connected, and outputs a blood glucose value generated by calibrating the received blood glucose biometric information to the user, and a terminal capable of communicating with the sensor transmitter (10), such as a smart phone, a tablet PC, or a notebook computer and so on, may be used as the communication terminal. Of course, the communication terminal (30) is not limited thereto, and may be any type of terminal as long as the terminal includes a communication function and a program or application can be installed to the terminal.

Here, the sensor transmitter (10) and the communication terminal (30) may be communicationally connected to each other by a wired connection way such as a USB cable or a wireless communication way such as infrared communication, NFC communication, Bluetooth and so on.

The communication terminal (30) needs calibration information to be used to calibrate blood glucose biometric information received from the sensor transmitter (10), and preferably, calibration information of the sensor is also obtained when a quality management code is read through the blood glucose management application and blood glucose biometric information is calibrated based on the acquired calibration information of the sensor.

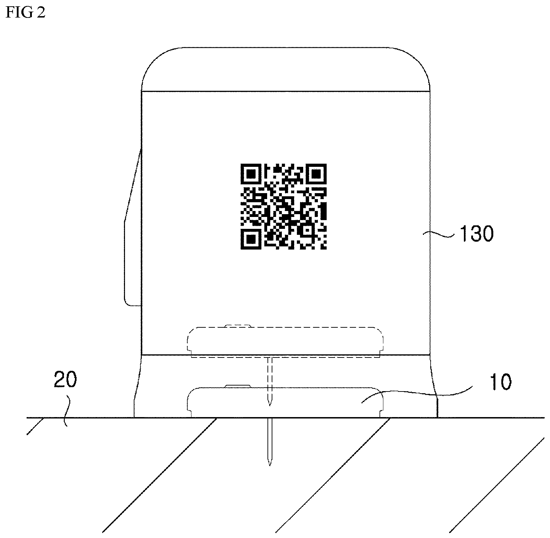

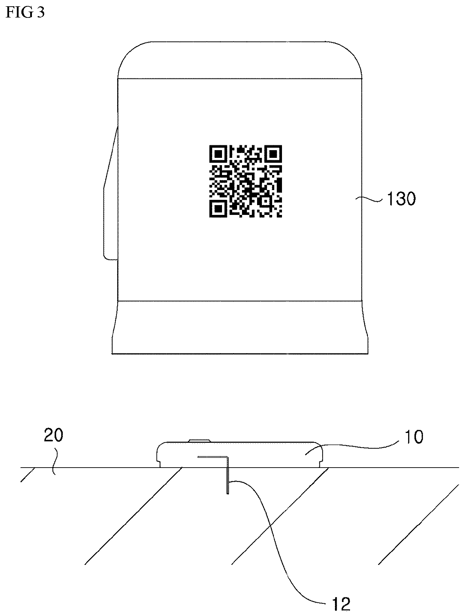

As described above, the sensor transmitter is attached to a part of the body through the applicator, and FIGS. 2 and 3 are diagrams for explaining a process of attaching a sensor transmitter to body using an applicator.

The applicator (130) has the sensor transmitter (10) therein and is configured to operate to discharge the sensor transmitter (10) to the outside of the applicator (130) by the user's manipulation to attach the sensor transmitter (10) to a specific body part of the user. The applicator (130) has a shape in which one side of the applicator (130) is opened, and the sensor transmitter (10) is installed to the applicator (130) through the open side of the applicator (130).

Looking at a process of attaching the sensor transmitter (10) to the body in more detail with reference to FIGS. 2 and 3, the open side of the applicator (130) adheres to a specific part of the body skin (20) in a state in which the protection cap is removed. When the applicator (130) is operated while the applicator (130) is in close contact with the skin (20) of the body, the sensor transmitter (10) may be discharged from the applicator (130) and is attached to the skin (20). Here, one end of a sensor (12) is arranged to be exposed from the sensor transmitter (10) at the lower part of the sensor transmitter (10), and one end of the sensor (12) is inserted into the skin (20) through a needle provided in the applicator (130). Accordingly, the sensor transmitter (10) may be attached to the skin (20) in a state in which one end of the sensor (12) is inserted to the skin (20).

Here, an adhesive tape may be provided on a body contact surface of the sensor transmitter (10) so that the sensor transmitter (10) can be fixedly attached to the skin (20) of the body. Therefore, when the applicator (130) is separated from the skin (20) of the body, the sensor transmitter (10) is fixedly attached to the skin (20) of the body by the adhesive tape.

After that, when power is supplied to the sensor transmitter (10), the sensor transmitter (10) connects the communication with the communication terminal (30), and the sensor transmitter (10) transmits the measured blood glucose information to the communication terminal.

The sensor transmitter (10) may measure not only blood glucose information but also various kinds of biometric information, but, hereinafter, blood glucose information will be described as an example of biometric information.

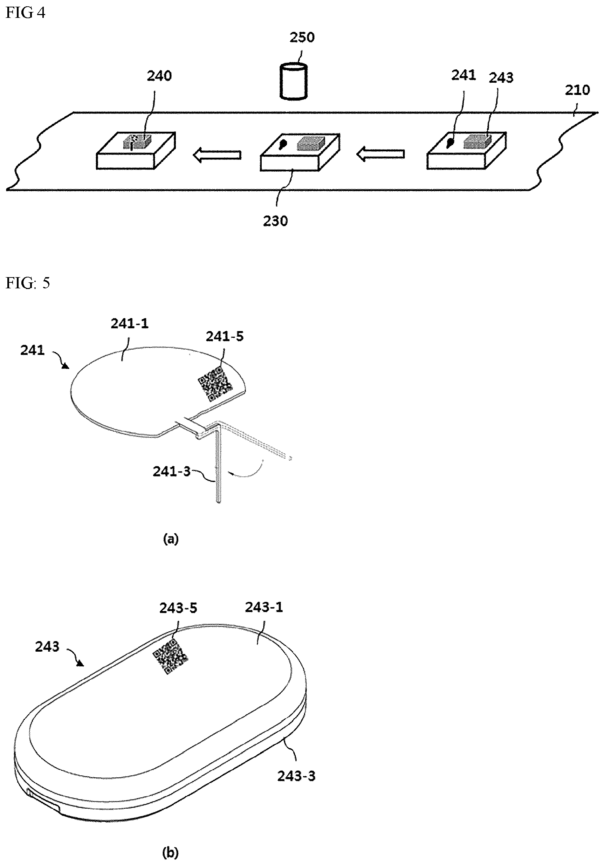

FIG. 4 is a diagram for explaining a process of manufacturing a sensor transmitter according to an embodiment of the present disclosure.

Referring to FIG. 4, a transfer belt (210) is driven in a factory manufacturing a sensor transmitter, and a plurality of trays (230) containing a sensor (241) for continuous blood glucose measurement and a transmitter (243) move along the transfer belt (210).

A reading camera (250) is disposed above the transport belt (210) and reads production information of the sensor (241) or the transmitter (243) contained in the tray (230) that moves along the transport belt (210). Depending on the field to which the present disclosure is applied, a portable reading device for reading the production information of the sensor (241) or the transmitter 243 by an operator may be used instead of the reading camera (250) disposed on the top of the conveying belt (210).

After obtaining the production information of the sensor (241) and the transmitter (243) contained in the tray (230) moving along the transfer belt (210), the operator assembles the sensor (241) and the transmitter (243) contained in the tray (230) to manufacture the sensor transmitter (240).

FIG. 5 shows an example of a sensor and a transmitter contained in the tray (230), as shown in FIG. 5(a), the sensor (241) is configured to have a first part (241-1) where an electrode connected to the transmitter is formed and a second part (241-3) inserted into the skin, and a sensor identifier (241-5) having production information of the sensor is formed at the first part (241-1). On the other hand, as shown in FIG. 5(b), the transmitter (243) has a housing comprising an upper housing (243-1) and a lower housing (243-3), and a sensor 241 is inserted into an inside of the housing and a plurality of components are provided for generating blood glucose biometric information from the body fluid extracted from the sensor and transmitting the blood glucose biometric information to the communication terminal. A transmitter identifier (243-5) having production information of the transmitter is formed on one surface of the upper housing (243-1) of the transmitter.

Referring back to FIG. 4, a manufacturing terminal (not shown) generates a quality management code by mapping the production information obtained from the sensor (241) are the production information obtained from the transmitter (243) contained in the tray (230) to each other, and the manufacturing terminal generates a quality management code having quality management information and then creates a label with the quality management code. Here, the label may be generated as a QR code, barcode, RFID, or the like. Here, the quality management code may be generated to include only the production information of the transmitter or both the production information of the transmitter and the production information of the sensor among the quality management information.

The manufacturer assembles the manufactured sensor transmitter (240) to the applicator (not shown), and after assembling the sensor transmitter (240) to the applicator, the generated label is attached to one surface of the applicator.

The label attached to the applicator may be re-generated in a subsequent process, such as a sterilization process, a packaging process, and so on, and the re-generated label is attached to a first packaging for sterilization, a second packaging for shipment, and so on and sold to the user.

After activating the blood glucose management application of the communication terminal, the user may acquire the production information of the sensor or obtain the production information of the transmitter from the label attached to the applicator, the first packaging, the second packaging for shipment, and so on, and blood glucose biometric information can be calibrated using the production information of the sensor or the communication with the communication terminal can be easily connected using the production information of the transmitter.

Here, when the manufacturing terminal (not shown) generates quality management information, the manufacturing terminal may transmit the quality management information to a manufacturer management server, and the manufacturer management server can monitor a distribution path or sales path of the sensor transmitter or use it for quality control of the sensor transmitter or easily tracks defective sensor transmitters based on the quality management information.

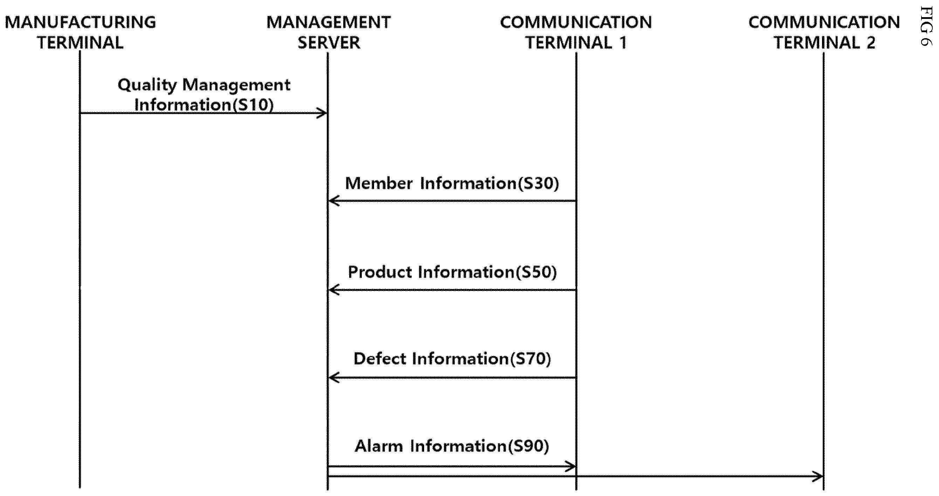

FIG. 6 is a diagram for explaining a message transmitted and received between a manufacturing terminal, a management server and a communication terminal according to an embodiment of the present disclosure.

Here, the manufacturing terminal is a terminal that is placed in a plant manufacturing sensor transmitters and is used to generate quality management information of the sensor transmitter, the management server is a server providing a blood glucose management application and processing a distribution route or performing defective product processing using the quality management information, and the manufacturing terminal, the management server, and the communication terminal are connected to each other through a wired or wireless network.

Looking more in detail with reference to FIG. 6, the manufacturing terminal that has generated quality management information during the manufacturing process of a sensor transmitter transmits the quality management information of the sensor transmitter to the management server (S10).

Sensor transmitters may be produced as sensors having different characteristics from each other in a plurality of factories, and the management server stores quality management information for sensor transmitters manufactured in each factory.

Preferably, the manufacturing terminal generates quality management information including production information of a sensor and production information of a transmitter, as well as time of manufacturing a sensor transmitter, a factory identifier of a factory manufacturing the sensor transmitter, and the like, and can transmit the generated quality management information to the management server.

The user may register as a member by transmitting member information for accessing the management server to the management server using the communication terminal (S30), and the user can register as a member by transmitting member information including the user's name, user identifier, age, address, nationality, contact information, and so on.

After that, when the user wants to use a new sensor transmitter, the blood glucose management application of the communication terminal is activated to obtain production information of a sensor or production information of a transmitter from a quality management code of a label attached to an applicator or a packaging, etc., and the obtained production information of the sensor or the obtained production information of the transmitter is transmitted together with the member information to the management server (S50).

The management server maps the production information of the sensor or the production information of the transmitter received from the communication terminal to the member information and store and manage them, and, by mapping the quality management information of the sensor transmitter stored in the management server and the member information actually using the sensor transmitter to each other and storing and managing them, monitors distribution paths or sales routes, such as countries in which sensor transmitters are used, or receives a reference blood glucose value measured by a user through a blood glucose meter and blood glucose biometric information measured through a continuous blood glucose measurement device together with quality management information of the sensor transmitter from the communication terminal, thereby managing the quality such as the accuracy of a sensor transmitter or easily tracking a defective sensor transmitter.

Afterwards, when a defect message of a defective sensor transmitter is received through the communication terminal (S70), alarm information for requesting to stop using a sensor transmitter can be transmitted based on the defect message to a communication terminal of a user who uses or purchases a sensor transmitter which uses the same sensor as the defective sensor transmitter of which defect message is received or is manufactured with a transmitter using the same component (S90). Based on the alarm information, the user can stop using a sensor transmitter being used or exchange a sensor transmitter purchased by the user for a new sensor transmitter.

Here, the defect message is transmitted through the communication terminal directly by the user at the user's request, or automatically to the management server if abnormality is detected by monitoring blood glucose biometric information measured in the communication terminal or failure in communication with the sensor transmitter and so on.

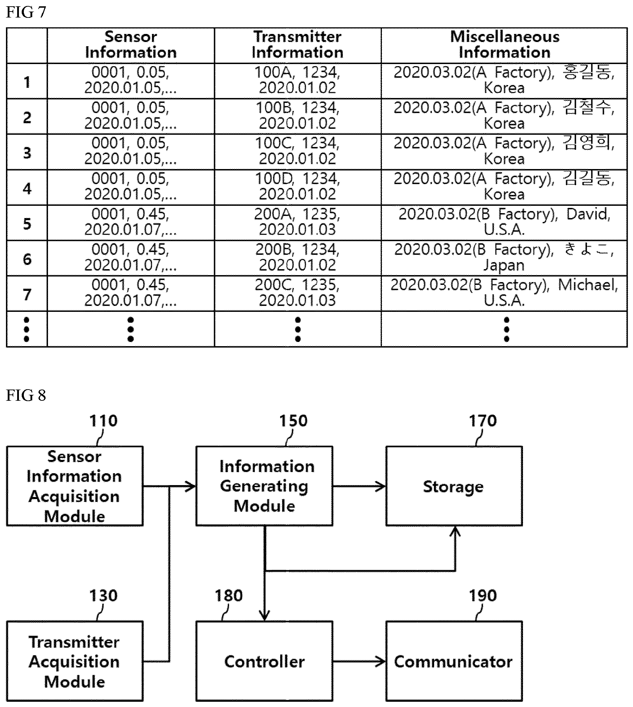

FIG. 7 shows an example of quality management information stored in a management server according to an embodiment of the present disclosure, and, referring to FIG. 7, sensor information, transmitter information and other information are mapped to each other and stored in the management server. The sensor information includes lot information (0001, 0002), calibration information (0.05, 0.45), a sensor manufacturing date of a corresponding sensor and so on, the transmitter information includes a unique serial number (100A, 100B, 100C, 100D, 200A, 200B, 200C) of a corresponding transmitter, a pin number (1234, 1234) of a corresponding transmitter, a manufacturing date of a corresponding transmitter and so on, and a factory identifier (Factory A, Factory B) of a factory where a sensor transmitter was manufactured, user information (name, nationality, address, etc.) of a user who is using or has purchased a transmitter are mapped to each other and stored in other information.

As such, information about the sensor, the transmitter, and the sensor transmitter are mapped to each other and stored in the management server, and for example, when defect regarding a sensor transmitter which a user HongGilDong is using is reported, alarm warning is transmitted to other users using the same sensor or transmitter, thereby reducing the damage caused by incorrect blood glucose measurement.

In addition, when it is necessary to change sensor calibration information of a sensor transmitter being used by a user, new calibration information may be transmitted to a communication terminal of a user using the same sensor to accurately monitor a blood glucose level.

In addition, a distribution route of a sensor transmitter or preference of a sensor transmitter for each nationality and each address can be easily tracked based on the nationality and address of a user who is using the sensor transmitter manufactured in each factory.

FIG. 8 is a functional block diagram for illustrating an example of a manufacturing terminal according to the present disclosure.

Referring to FIG. 8 in more detail, a sensor information acquisition module (110) acquires sensor production information from a sensor for continuous blood glucose measurement contained in a tray moving along a transport belt. Here, the sensor information acquisition module (110) may use a camera that reads a sensor identifier formed on the sensor, and various reading devices for reading the sensor identifier may be used depending on types of sensor identifiers according to the field to which the present disclosure is applied.

The transmitter acquisition module (130) acquires transmitter production information from the transmitter contained in the tray moving along the transfer belt. Here, the transmitter acquisition module (130) may use a camera that reads a transmitter identifier formed at the transmitter, and various reading devices for reading a transmitter identifier may be used according to types of transmitter identifiers according to the field to which the present disclosure is applied. For example, the production information of the transmitter may be stored in the memory of the transmitter, and the transmitter acquisition module (130) may be connected to the transmitter by wire or wirelessly to obtain the production information of the transmitter from the memory of the transmitter.

When acquiring the production information of the sensor and the production information of the transmitter, an information generating module (150) maps the production information of the sensor and the production information of the transmitter to each other to generate quality management information, and the generated quality management information is stored in a storage (170). Preferably, the information generating module (150) may generate a quality management code capable of reading quality management information, and provide a label including the quality management code to a separate label generating module (not shown). The label generating module may generate a label so that only production information of the transmitter can be read from the quality management code among the quality management information or both the production information of the transmitter and the production information of the sensor can be read from the quality management code.

When a cycle set for quality management information stored in the storage (170) reaches or when the number of quality management information stored in the storage (170) reaches a set number by counting the number of quality management information stored in the storage (170), the quality management information is transmitted to the management server through the communicator (190). Preferably, the controller (180) may include the manufacturing date of a sensor transmitter, a factory identifier of a factory which has manufactured the sensor transmitter and so on in the quality management information and transmit the quality management information to the management server.

Preferably, the controller (180) may control to store the generated quality management information in the memory of the transmitter. Production information of the sensor may be stored in a first area of the memory of the transmitter, and production information of the transmitter may be stored separately from the production information of the sensor in a second area of the memory of the transmitter.

Therefore, the user can directly obtain the production information of the sensor and the production information of the transmitter by reading the label through the blood glucose management application of the communication terminal, but after the production information of the transmitter, for example, a serial number, a pin code and so on, is acquired by reading the label to connect the communication between the sensor transmitter and the communication terminal, the production information of the sensor stored in the memory of the transmitter, for example, calibration information, lot information, production date, etc., can be automatically received from the sensor transmitter.

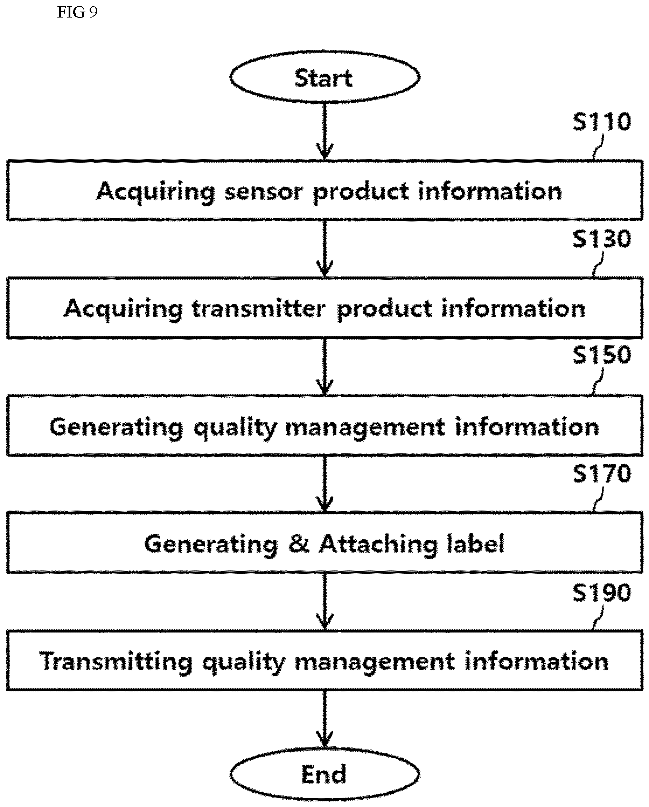

FIG. 9 is a flowchart for explaining a method of manufacturing a sensor transmitter according to an embodiment of the present disclosure.

Referring to FIG. 9, production information of the sensor is obtained from a sensor (S110). Preferably, the production information of the sensor can be obtained through a sensor identifier formed at the sensor, and the production information of the sensor may include lot information including characteristics of the sensor, a factory of manufacturing the sensor, manufacturing date of the sensor, calibration information of the sensor, and so on.

The production information of the transmitter is acquired from the transmitter (S130). Preferably, the production information of the transmitter may be obtained through a transmitter identifier formed at the transmitter or through a memory of the transmitter, and the production information of the transmitter includes a factory of manufacturing the transmitter, manufacturing date of the transmitter, a serial number of the transmitter, a pin number of the transmitter, production date of the transmitter, and so on.

Quality management information of the sensor transmitter is generated by mapping the acquired production information of the sensor and the acquired production information of the transmitter to each other (S150). When the quality management information is generated, at least one label having a quality management code indicating the quality management information is generated according to the manufacturing process of the sensor transmitter, and the generated label is attached to the applicator or the packaging according to the manufacturing process of the sensor transmitter (S170).

On the other hand, the generated sensor transmitter quality management information is transmitted to the management server (S190).

Meanwhile, the exemplary embodiments of the present disclosure described above can be implemented through programs executable at computers, and can be operated in a general-purpose digital computer executing the programs using computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

Although the present disclosure is described with reference to embodiments shown in the drawings in order to explain certain principles of the present disclosure by way of example, a person having ordinary skill in the art which the present disclosure relates could make various modifications and equivalent other embodiments. Accordingly, the protection scope of the present invention shall be defined by the claims attached hereto and all of their equivalents.

## Claims

1. A method of manufacturing a sensor transmitter (240) for continuous blood glucose measurement, the method comprising:
acquiring product information of a sensor (241) disposed in a tray (230);
acquiring product information of a transmitter (243) disposed in the tray (230) moving along a transfer belt (210);
generating quality management information by mapping the acquired product information of the sensor (241) and the acquired product information of the transmitter (243) to each other; and
transmitting the quality management information to a server to store and manage the quality management information in the server,
wherein the sensor (241) and the transmitter (243) are disposed in one same tray (230), and the sensor (241) and the transmitter (243) disposed in the same tray (230) are assembled to manufacture the sensor transmitter (240) after generation of the quality management information,
wherein the quality management information includes time of manufacturing a sensor transmitter.

2. The method of manufacturing the sensor transmitter (240) according to claim 1, further comprising:
generating a quality management code used to read the quality management information, and
generating a label including the generated quality management code.

3. The method of manufacturing the sensor transmitter (240) according to claim 2,
wherein the quality management code is used to be capable of reading the production information of the transmitter (243) only among the quality management information, or is used to be capable of reading both the production information of the transmitter (243) and the production information of the sensor (241).

4. The method of manufacturing the sensor transmitter (240) according to claim 2 or 3, wherein the label is attached to at least one of an applicator (130) to which the sensor transmitter is mounted, an inner packaging of the applicator (130), and an outer packaging of the applicator (130).

5. The method of manufacturing the sensor transmitter (240) according to any one of claims 2 to 4, further comprising storing the quality management information to memory of the transmitter (243),
wherein the production information of the transmitter among the quality management information is stored to a first area of the memory, and the production information of the sensor (241) among the quality management information is stored to a second area of the memory.

6. The method of manufacturing the sensor transmitter (240) according to any one of claims 1 to 5, wherein the production information of the sensor (241) is acquired by reading a sensor identifier formed on the sensor.

7. The method of manufacturing the sensor transmitter (240) according to any one of claims 1 to 5, wherein the production information of the transmitter is acquired by reading a transmitter identifier formed on an outer housing of the transmitter.

8. The method of manufacturing the sensor transmitter (240) according to any one of claims 1 to 5, wherein the production information of the transmitter (243) is stored in the memory of the transmitter, and the production information of the transmitter is acquired from the memory.

## Patentansprüche

1. Verfahren zur Herstellung eines Sensorsenders (240) zur kontinuierlichen Blutzuckermessung, wobei das Verfahren umfasst:
Erfassen von Produktinformationen eines Sensors (241), der in einer Schale (230) angeordnet ist;
Erfassen von Produktinformationen eines Senders (243), der in der Schale (230) angeordnet ist, die sich entlang eines Förderbands (210) bewegt;
Generieren von Qualitätsmanagementinformationen durch Verknüpfen der erfassten Produktinformationen des Sensors (241) und der erfassten Produktinformationen des Senders (243) miteinander; und
Übermitteln der Qualitätsmanagementinformationen an einen Server, um die Qualitätsmanagementinformationen in dem Server zu speichern und zu verwalten,
wobei der Sensor (241) und der Sender (243) in ein und derselben Schale (230) angeordnet sind und der Sensor (241) und der Sender (243), die in derselben Schale (230) angeordnet sind, nach der Generierung der Qualitätsmanagementinformationen zusammengesetzt werden, um den Sensorsender (240) herzustellen,
wobei die Qualitätsmanagementinformationen den Zeitpunkt der Herstellung eines Sensorsenders beinhalten.

2. Verfahren zur Herstellung des Sensorsenders (240) nach Anspruch 1, weiterhin umfassend:
Generieren eines Qualitätsmanagementcodes, der zum Auslesen der Qualitätsmanagementinformationen verwendet wird, und
Generieren eines Labels, das den generierten Qualitätsmanagementcode enthält.

3. Verfahren zur Herstellung des Sensorsenders (240) nach Anspruch 2, wobei der Qualitätsmanagementcode so verwendet wird, dass nur die Produktionsinformationen des Senders (243) unter den Qualitätsmanagementinformationen ausgelesen werden können, oder so verwendet wird, dass sowohl die Produktionsinformationen des Senders (243) als auch die Produktionsinformationen des Sensors (241) ausgelesen werden können.

4. Verfahren zur Herstellung des Sensorsenders (240) nach Anspruch 2 oder 3, wobei das Label an zumindest einem von einem Applikator (130), an dem der Sensorsender montiert ist, einer Innenverpackung des Applikators (130) und einer Außenverpackung des Applikators (130) angebracht ist.

5. Verfahren zur Herstellung des Sensorsenders (240) nach einem der Ansprüche 2 bis 4, weiterhin umfassend das Speichern der Qualitätsmanagementinformationen in einem Speicher des Senders (243),
wobei die Produktionsinformationen des Senders unter den Qualitätsmanagementinformationen in einem ersten Bereich des Speichers gespeichert werden und die Produktionsinformationen des Sensors (241) unter den Qualitätsmanagementinformationen in einem zweiten Bereich des Speichers gespeichert werden.

6. Verfahren zur Herstellung des Sensorsenders (240) nach einem der Ansprüche 1 bis 5, wobei die Produktionsinformationen des Sensors (241) durch Auslesen einer auf dem Sensor gebildeten Sensorkennung erfasst werden.

7. Verfahren zur Herstellung des Sensorsenders (240) nach einem der Ansprüche 1 bis 5, wobei die Produktionsinformationen des Senders erfasst werden, indem eine auf einem Außengehäuse des Senders ausgebildeten Senderkennung ausgelesen wird.

8. Verfahren zur Herstellung des Sensorsenders (240) nach einem der Ansprüche 1 bis 5, wobei die Produktionsinformationen des Senders (243) in dem Speicher des Senders gespeichert sind und die Produktionsinformationen des Senders aus dem Speicher erfasst werden.

## Revendications

1. Procédé de fabrication d'un émetteur de capteur (240) pour la mesure continue de glycémie, ledit procédé comprenant :
l'acquisition d'informations de produit d'un capteur (241) disposé dans un support (230) ;
l'acquisition d'informations d'un émetteur (243) disposé dans le support (230) se déplaçant le long d'une bande de transfert (210) ;
la génération d'informations de gestion de qualité par mise en correspondance des informations de produit acquises du capteur (241) avec les informations de produit acquises de l'émetteur (243) ; et
la transmission des informations de gestion de qualité à un serveur pour le stockage et la gestion desdites informations de gestion de qualité dans le serveur,
où le capteur (241) et l'émetteur (243) sont disposés dans un même support (230), et le capteur (241) et l'émetteur (243) disposés dans le même support (230) sont assemblés pour fabriquer émetteur de capteur (240) après génération des informations de gestion de qualité,
où les informations de gestion de qualité comprennent le temps de fabrication d'un émetteur de capteur.

2. Procédé de fabrication d'un émetteur de capteur (240) selon la revendication 1, comprenant en outre :
la génération d'un code de gestion de qualité utilisé pour lire les informations de gestion de qualité, et
la génération d'une étiquette comprenant le code de gestion de qualité généré.

3. Procédé de fabrication d'un émetteur de capteur (240) selon la revendication 2, où le code de gestion de qualité est utilisé pour pouvoir lire les informations de production de l'émetteur (243) uniquement parmi les informations de gestion de qualité, ou est utilisé pour pouvoir relire à la fois les informations de production de l'émetteur (243) et les informations de production du capteur (241).

4. Procédé de fabrication d'un émetteur de capteur (240) selon la revendication 2 ou la revendication 3, où l'étiquette est fixée à un applicateur (130) sur lequel l'émetteur de capteur est monté et/ou à un emballage intérieur de l'applicateur (130) et/ou à un emballage extérieur de l'applicateur (130).

5. Procédé de fabrication d'un émetteur de capteur (240) selon l'une des revendications 2 à 4, comprenant en outre le stockage des informations de gestion de qualité dans la mémoire de l'émetteur (243),
où les informations de production de l'émetteur parmi les informations de gestion de qualité sont stockées dans une première zone de la mémoire, et les informations de production du capteur (241) parmi les informations de gestion de qualité sont stockées dans une deuxième zone de la mémoire.

6. Procédé de fabrication d'un émetteur de capteur (240) selon l'une des revendications 1 à 5, où les informations de production du capteur (241) sont acquises par lecture d'un identifiant de capteur formé sur le capteur.

7. Procédé de fabrication d'un émetteur de capteur (240) selon l'une des revendications 1 à 5, où les informations de production de l'émetteur sont acquises par lecture d'un identifiant d'émetteur formé sur un boîtier extérieur de l'émetteur.

8. Procédé de fabrication d'un émetteur de capteur (240) selon l'une des revendications 1 à 5, où les informations de production de l'émetteur (243) sont stockées dans la mémoire de l'émetteur, et les informations de production de l'émetteur sont acquises à partir de la mémoire.
